Europäisches Patentamt

European Patent Office

Office européen des brevets

(11) Numéro de publication: **0 369 880**
**A1**

## (12) DEMANDE DE BREVET EUROPEEN

(21) Numéro de dépôt: 89403141.8

(51) Int. Cl.5: **A61K 9/06, A61K 45/06**

(22) Date de dépôt: **15.11.89**

| | |
|---|---|
| Revendications pour les Etats contractants suivants: ES + GR | (71) Demandeur: **CORBIERE, Jérôme**<br>**17, rue Cortambert**<br>**F-75116 Paris(FR)** |
| (30) Priorité: **16.11.88 FR 8814907** | |
| (43) Date de publication de la demande:<br>**23.05.90 Bulletin 90/21** | (72) Inventeur: **CORBIERE, Jérôme**<br>**17, rue Cortambert**<br>**F-75116 Paris(FR)** |
| (84) Etats contractants désignés:<br>**AT BE CH DE ES FR GB GR IT LI LU NL SE** | (74) Mandataire: Burtin, **Jean-François**<br>**Bugnion Associés Département GEF1B 55**<br>**Rue Boissonade**<br>**F-75014 Paris(FR)** |

(54) **Nouvelles compositions pharmaceutiques agissant sur la presbytie et leur procédé d'obtention.**

(57) La présente invention se rapporte au domaine de la chimie thérapeutique et notamment à de nouvelles compositions pharmaceutiques.

Elle a plus particulièrement pour objet des compositions pharmaceutiques renfermant à titre de principe actif un agent spasmolytique du type musculotrope ou un agent ganglioplégique du type musculaire, en association ou en mélange avec un véhicule aqueux approprié pour l'administration par voie oculaire.

Les compositions pharmaceutiques selon l'invention servent de médicament pour limiter, retarder ou supprimer les effets de la presbytie.

EP 0 369 880 A1

# NOUVELLES COMPOSITIONS PHARMACEUTIQUES AGISSANT SUR LA PRESBYTIE ET LEUR PROCEDE D'OBTENTION

La présente invention se rapporte à de nouvelles compositions pharmaceutiques destinées à limiter, à retarder ou à supprimer les effets de la presbytie.

Elle a plus particulièrement pour objet des compositions pharmaceutiques renfermant à titre de principe actif, un agent spasmolytique du type musculotrope ou un agent ganglioplégique du type musculaire, en association ou en mélange avec un véhicule aqueux approprié pour l'administration par voie oculaire.

L'amplitude de l'accommodation diminue progressivement avec l'âge. A l'âge de 8 ans, la capacité dioptrique de l'oeil normal peut augmenter par accommodation jusqu'à 14 d. A l'âge de 20 ans, cette capacité tombe à 11 D et à l'âge de 50 ans, elle est inférieure à 2 D. Le point le plus proche pour lequel l'oeil peut accommoder de façon à ce qu'une image claire soit formée sur la rétine, est appelé le point proche ou punctum proximum, si l'oeil est immétropique ou est formé de cette façon avec des lentilles correctives appropriées, le punctum proximum est susceptible de varier avec l'âge du sujet. Une perte supplémentaire du pouvoir d'accommodation avec l'éloignement du functum proximum se poursuit sans cesse jusqu'à l'âge de 60 ans environ et à peu près à cette période, toute la capacité d'accommodation a disparu (Adler's Physiology of the eye - Ed. C.V Mosby Company ST LOUIS - 1975).

On sait que la presbytie est le résultat d'une sclérose ou tout au moins, d'un durcissement du noyau du cristallin de sorte que les forces musculaires qui normalement déforment le cristallin souple pendant la jeunesse (élasticité capsulaire) deviennent dépourvues d'efficacité. On a également suggéré que la diminution de la plasticité du cortex résultait d'une diminution de la teneur en eau du cristallin du fait de l'âge. On a trouvé que la surface du cristallin après ablation est plus fortement incurvée chez les sujets âgés que chez les enfants et ceci implique que l'élasticité de la capsule n'est désormais plus en mesure de déformer le cristallin chez le sujet âgé.

En outre la chirurgie peut corriger dans une certaine mesure le défaut de convexité du cristallin mais ne rétablit que pendant un certain temps une vision normale des objets.

La présente invention vise à remédier à cet état de choses en apportant une solution thérapeutique au traitement de la presbytie.

On a constaté, en effet, que l'instillation dans l'oeil de solutions d'agents spasmolytiques du type musculotrope, améliorait sensiblement le degré de vision et permettait d'obtenir ou de retrouver progressivement une vision quasiment normale.

On peut définir la classe des antispasmodiques musculotropes comme une famille de composés, diverse sur le plan chimique mais individualisée sur le plan pharmacologique, principalement par son absence d'effet du type atropinique et par son aptitude à lever le spasme expérimental induit par le chlorure de Baryum (cf. J. Frexinos Med. Chir. Digest. 16 (1987) 567). Dans cette famille on pourra citer plus particulièrement les molécules phénoliques comme le phloroglucinol ou l'Alibendol, des ammoniums quaternaires non cholinergiques comme le bromure de Pinaverium, des esters aromatiques d'amino-alcools comme la dihexyverine, le chlorhydrate de Mebeverine, la Dipropyline, des esters phenyl acétiques comme la Camilofine, des phenothiazines comme la fenoverine, des alcaloïdes isoquinoléiniques comme la Papavérine, l'Ethavérine ou l'Octavérine, des butyrophénones comme la Pitofénone, des diphénylpropyl-amines comme l'Alvérine ou bien encore des agonistes enkephaliniques comme la Trimébutine.

Cette liste n'est pas limitative et est susceptible d'englober pratiquement tous les composés répondant à la définition d'agent spasmolytique ou ganglioplegique musculotrope.

Cette définition exclut par contre les produits à action mydriatique càmme l'Atropine et ses dérivés, les sels d'ammonium quaternaire dérivés de l'Hyoscine et les sels d'ammonium quaternaire à longue chaine, anti-cholinestérasiques qui ont une action neurotrope plus que musculotrope.

Les compositions pharmaceutiques selon l'invention renferment donc au moins un dérivé spasmolytique musculotrope ou ganglioplégique en association ou en mélange avec un véhicule aqueux isotonique aux larmes qui contient en outre si nécessaire, un agent antibactérien, un agent stabilisant ou chelatant, un agent colorant.

Comme agent isotonisant, on citera plus particulièrement les sels de sodium, de lithium ou de magnésium comme le chlorure de sodium ou l'acétate de lithium.

Comme agent antibactérien, on citera plus particulièrement des organomercuriels comme le thimerosal ou le nitrate de phényl mercure, des parabens comme le p.hydroxybenzoate de méthyle ou de propyle, des ammoniums quaternaires comme les sels de cetrimonium ou de benzalkonium.

Comme agent stabilisant, on citera plus particulièrement les bisulfites alcalins, les métabisulfites alcalins, les hypophosphites alcalins et les dérivés de l'acide ascorbique.

Comme agent chelatant, on citera en particulier les sels de l'acide éthylène-diaminotétra-acétique, ceux de l'acide éthylèneglycol bis-(β-aminoétyléther)-N,N,N′,N′-tétra-acétique, de l'acide éthylènediamine-N,N′-diacétique-N,N′-di-α-propionique ou de l'acide éthylène-diamino-N,N′-diacétique-N,N′-di-β-propionique. Ces complexes peuvent plus particulièrement être constitués par des molécules mixtes renfermant deux ou quatre ions alcalins.

Les solutions selon l'invention peuvent en outre être additionnées d'agent épaississant ou augmentant la viscosité, d'agents édulcorants et/ou d'agents parfumants.

Les solutions selon l'invention sont destinées à être administrées sous forme de collyres ou d'instillations oculaires. D'une manière préférée, on utilise des solutions isotoniques renfermant de 0,1 % à 1 % de principe actif et plus précisément de 0,25 à 0,5 ‰. La posologie moyenne est de 1 à 4 administrations quotidiennes pendant une période s'échelonnant de 1 à 3 mois.

L'invention a également pour objet un procédé d'obtention des compositions oculaires selon l'invention qui consiste en ce que le principe actif du type antispasmodique musculotrope ou ganglioplégique est dissout dans un véhicule aqueux et additionné d'un ou plusieurs adjuvants pharmaceutiques appropriés pour usage par voie oculaire et d'un agent isotonisant.

Les exemples suivants illustrent l'invention. Ils ne la limitent en aucune façon :

## EXEMPLE I

| Collyre au bromure de Pinaverium à 0,1 % | |
|---|---|
| Bromure de Pinaverium ... | 1 g |
| Phosphate monosodique hydraté avec 2 mol d'eau ... | 1 g |
| Phosphate disodique hydraté avec 12 mol d'eau ... | 9,38 g |
| Chlorure de Benzalkonium ... | 18,48 mg |
| Hypophosphite de sodium ... | 0,10 g |
| Eau stérile Q.S.P ... | 1000 ml |

Les composants préalablement stérilisés sont ajoutés à l'eau stérile et mis en solution. Le pH de la suspension est ajusté à 6,8 en atmosphère stérile et la solution est finalement diluée au volume requis puis répartie stérilement en doses unitaires de 2 ml.

## EXEMPLE II

| Collyre à la Trimébutine à 0,15 % | |
|---|---|
| Trimébutine (chlorhydrate) ... | 1,61 g |
| Phosphate monosodique à 2 mol d'eau ... | 0,64 g |
| Phosphate disodique à 12 mol d'eau ... | 1,68 g |
| Nitrate de phénylmercure ... | 0,10 g |
| Carboxyméthyl cellulose ... | 0,15 g |
| Eau stérile Q.S.P ... | 1000 ml |

## EXEMPLE III

| Collyre à l'octavérine à 0,5 % | |
| --- | --- |
| Octavérine (chlorhydrate) ... | 0,535 g |
| Acétate de sodium ... | 0,042 g |
| Ascorbate de sodium ... | 0,5 g |
| p.hydroxybenzoate de méthyle ... | 0,045 g |
| p.hydroxybenzoate de propyle ... | 0,045 g |
| Hydroxypropyl méthyl cellulose ... | 0,030 g |
| Tétracémate disodique ... | 0,025 g |
| Eau stérile Q.S.P ... | 100 ml |

## EXEMPLE IV

| Collyre au chlorhydrate de Mébéverine à 0,2 % | |
| --- | --- |
| Chlorhydrate de Mébéverine ... | 0,21 g |
| Chlorure de lithium ... | 0,27 g |
| Hydroxypropyl méthyl cellulose ... | 0,02 g |
| Ascorbate de sodium ... | 0,35 g |
| Eau de rose q.s | |
| Eau stérile Q.S.P ... | 100 ml |

## Revendications

1°- Utilisation d'un principe actif choisi dans le groupe constitué par un agent spasmolytique du type musculotrope et un agent ganglioplégique du type musculaire en vue de la réalisation d'un médicament ophtalmique destiné à limiter, retarder ou supprimer les effets de la presbytie.

2°- Utilisation d'un principe actif choisi dans le groupe constitué par un agent spasmolytique du type musculotrope et un agent ganglioplégique du type musculaire selon la revendication 1° dans laquelle le principe actif est un agent antispasmodique musculotrope du type phénolique.

3°- Utilisation d'un principe actif choisi dans le groupe constitué par un agent spasmolytique du type musculotrope et un agent ganglioplégique du type musculaire selon la revendication 1° dans laquelle le principe actif est un agent antispasmodique du type ammonium quaternaire non cholinergique.

4°- Utilisation d'un principe actif choisi dans le groupe constitué par un agent spasmolytique du type musculotrope et un agent ganglioplégique du type musculaire selon la revendication 1° dans laquelle le principe actif est un alcaloïde isoquinoléinique choisi parmi la Papaverine, l'Ethaverine et l'Octavérine.

5°- Utilisation d'un principe actif choisi dans le groupe constitué par un agent spasmolytique du type musculotrope et un agent ganglioplégique du type musculaire selon la revendication 1° dans laquelle le principe acctif est un agoniste enképhalinergique.

6°- Utilisation d'un principe actif choisi dans le groupe constitué par un agent spasmolytique du type musculotrope et un agent ganglioplégique du type musculaire selon la revendication 1° dans laquelle le principe actif est un ester aromatique d'amino alcool.

7°- Utilisation d'un principe actif choisi dans le groupe constitué par un agent spasmolytique du type musculotrope et un agent ganglioplégique du type musculaire selon la revendication 1° dans laquelle, le principe actif est une butyrophénone.

8°- Utilisation d'un principe actif choisi dans le groupe constitué par un agent spasmolytique du type musculotrope et un agent ganglioplégique du type musculaire selon l'une des revendications 1° à 7° dans laquelle la teneur en principe actif varie de 0,1 à 1%.

9°- Utilisation d'un principe acctif choisi dans le groupe constitué par un agent spasmolytique du type musculotrope et un agent ganglioplégique du type musculaire selon la revendication 8° dans laquelle la

4

teneur en principe actif varie de 0,25 à 0,5%.

10°- Un procédé d'obtention d'un médicament ophtalmique destiné à limiter, retarder ou supprimer les effets de la presbytie, selon l'une des revendications 1 à 9° qui consiste en ce qu'un principe actif choisi dans le groupe constitué par un agent anti-spasmodique musculotrope et un agent ganglioplégique du type musculaire est dissout dans un véhicule aqueux et additionné d'un ou plusieurs adjuvants pharmaceutiques Revendiétioosr busabes Èatsocentrababetstsduanésgeñsi©ñnisant.

1°- Un procédé d'obtention d'un médicament ophtalmique destiné à limiter, retarder ou supprimer les effets de la presbytie, qui consiste en ce qu'un principe actif choisi dans le groupe constitué par un agent anti-spasmodique musculotrope et un agent ganglioplégique du type musculaire est dissout dans un véhicule aqueux et additionné d'un ou plusieurs adjuvants pharmaceutiques appropriés pour l'usage par voie oculaire et d'un agent isotonisant.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-4 390 542 (R.A. SCHACHAR) <br> * Revendications 1-4 * <br> --- | 1,4,8,9 ,10 | A 61 K 9/06 <br> A 61 K 45/06 |
| X | M. NEGWER: "Organic-chemical drugs and their synonyms", vol. II, édition 6, 1987, pages 1024,1154,1180, Okadema Verlag, DE <br> * No. 5856: "difropyline"; no. 6530: "pitofenone hydrochloride"; no. 6665: "trimebutine" * <br> --- | 1,5-10 | |
| X | "The Merck Index", édition 10, 1983, pages 38,821,1072, Merck & Co. Inc., Rahway, NY, US <br> * No. 230: "alibendol"; no. 5590: "mebeverine"; no. 7315: "pinaverium bromide" * <br> --- | 1,2,3,6 ,8,9,10 | |
| X | EMBASE 81004169: F. REGNAULT: "Conseils pratiques en ophtamologie", & GAZ. MED. FR., 1980, 87/28, pages 3527-3530 <br> * Résumé * <br> ----- | 1 | DOMAINES TECHNIQUES RECHERCHES (Int. Cl.5) <br><br> A 61 K |

Le présent rapport a été établi pour toutes les revendications

| Lieu de la recherche | Date d'achèvement de la recherche | Examinateur |
|---|---|---|
| LA HAYE | 21-02-1990 | PEETERS J.C. |